# EUROPEAN PATENT APPLICATION

(11) **EP 0 835 879 A2**
(43) Date of publication of application: **15.04.1998**
(21) Application number: 97308003.9
(22) Date of filing: 09.10.1997
(51) Int. Cl.: C07K 14/575, A61K 38/22

(54) **Analogs of obesity proteins and pharmaceutical formulations comprising them**

(30) Priority: 11.10.1996 US 28335 P
(71) Applicant: ELI LILLY AND COMPANY, Indianapolis, Indiana 46285 (US)
(72) Inventor: Beals, John Michael, Indianapolis, Indiana 46278-1853 (US); Dimarchi, Richard Dennis, Carmel, Indiana 46033 (US)
(74) Representative: Denholm, Anna Marie

(57) **Abstract**

The present invention discloses a new class of anti-obesity proteins based on chimpanzee leptin. Soluble parenteral formulations comprising these proteins and methods of treating obesity are claimed.

SEQUENCE LISTING

(1) GENERAL INFORMATION:

(i) APPLICANT: ELI LILLY AND COMPANY
(B) STREET: Lilly Corporate Center
(C) CITY: Indianapolis
(D) STATE: Indiana
(E) COUNTRY: United States of America
(F) ZIP: 46285

(ii) TITLE OF INVENTION: Therapeutic Proteins

(iii) NUMBER OF SEQUENCES: 13

(iv) CORRESPONDENCE ADDRESS:
(A) ADDRESSEE: A. M. Denholm
(B) STREET: Erl Wood Manor
(C) CITY: Windlesham
(D) STATE: Surrey
(E) COUNTRY: United Kingdom
(F) ZIP: GU20 6PH

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30

(vi) CURRENT APPLICATION DATA:
(A) APPLICATION NUMBER: 97308003.9
(B) FILING DATE: 9th October 1997

(2) INFORMATION FOR SEQ ID NO:1:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:1:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Xaa Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Met Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Trp Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO:2:

    (i) SEQUENCE CHARACTERISTICS:
        (A) LENGTH: 146 amino acids
        (B) TYPE: amino acid
        (C) STRANDEDNESS: single
        (D) TOPOLOGY: linear

    (ii) MOLECULE TYPE: protein

    (xi) SEQUENCE DESCRIPTION: SEQ ID NO:2:

Val Pro Ile Gln Lys Val Gln Asp Asp Thr Lys Thr Leu Ile Lys Thr
1               5                   10                  15

Ile Val Thr Arg Ile Asp Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Met Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Trp Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys

145

(2) INFORMATION FOR SEQ ID NO:3:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:3:

Val Pro Ile Gln Lys Val Gln Asp Asp Thr Lys Thr Leu Ile Lys Thr
1               5                   10                  15

Ile Val Thr Arg Ile Asn Asp Ile Ser His Ala Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Met Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Trp Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO:4:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:4:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Xaa Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Met Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Trp Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO:5:

    (i) SEQUENCE CHARACTERISTICS:
        (A) LENGTH: 146 amino acids
        (B) TYPE: amino acid
        (C) STRANDEDNESS: single
        (D) TOPOLOGY: linear

    (ii) MOLECULE TYPE: protein

    (xi) SEQUENCE DESCRIPTION: SEQ ID NO:5:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Met Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Trp Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO:6:

    (i) SEQUENCE CHARACTERISTICS:
        (A) LENGTH: 146 amino acids
        (B) TYPE: amino acid
        (C) STRANDEDNESS: single
        (D) TOPOLOGY: linear

    (ii) MOLECULE TYPE: protein

    (xi) SEQUENCE DESCRIPTION: SEQ ID NO:6:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Met Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Ala Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO:7:

    (i) SEQUENCE CHARACTERISTICS:
        (A) LENGTH: 146 amino acids

(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:7:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Met Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Gln Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO:8:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:8:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Met Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Trp Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Gln Gln Leu Asp Leu Ser Pro
    130                 135                 140

Gly Cys
145

(2) INFORMATION FOR SEQ ID NO:9:

    (i) SEQUENCE CHARACTERISTICS:
        (A) LENGTH: 146 amino acids
        (B) TYPE: amino acid
        (C) STRANDEDNESS: single
        (D) TOPOLOGY: linear

    (ii) MOLECULE TYPE: protein

    (xi) SEQUENCE DESCRIPTION: SEQ ID NO:9:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Met Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Gln Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Gln Gln Leu Asp Leu Ser Pro
    130                 135                 140

Gly Cys
145

(2) INFORMATION FOR SEQ ID NO:10:

    (i) SEQUENCE CHARACTERISTICS:
        (A) LENGTH: 146 amino acids
        (B) TYPE: amino acid
        (C) STRANDEDNESS: single
        (D) TOPOLOGY: linear

    (ii) MOLECULE TYPE: protein

    (xi) SEQUENCE DESCRIPTION: SEQ ID NO:10:

Val Pro Ile Gln Lys Val Gln Asp Asp Thr Lys Thr Leu Ile Lys Thr
1               5                   10                  15

Ile Val Thr Arg Ile Asn Asp Ile Ser His Ala Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Met Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Ala Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO:11:

    (i) SEQUENCE CHARACTERISTICS:
        (A) LENGTH: 146 amino acids
        (B) TYPE: amino acid
        (C) STRANDEDNESS: single
        (D) TOPOLOGY: linear

    (ii) MOLECULE TYPE: protein

(xi) SEQUENCE DESCRIPTION: SEQ ID NO:11:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Met Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Ala Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Gln Gln Leu Asp Leu Ser Pro
    130                 135                 140

Gly Cys
145

(2) INFORMATION FOR SEQ ID NO:12:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:12:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Met Ile Gln Ile Ser Asn Asp Leu
65                  70                  75                  80

Glu Asn Leu Arg Asp Leu Leu His Val Leu Ala Phe Ser Lys Ser Cys
                85                  90                  95

Ser Leu Pro Gln Thr Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Gln Gln Leu Asp Leu Ser Pro
    130                 135                 140

Gly Cys
145

(2) INFORMATION FOR SEQ ID NO:13:

    (i) SEQUENCE CHARACTERISTICS:
        (A) LENGTH: 146 amino acids
        (B) TYPE: amino acid
        (C) STRANDEDNESS: single
        (D) TOPOLOGY: linear

    (ii) MOLECULE TYPE: protein

    (xi) SEQUENCE DESCRIPTION: SEQ ID NO:13:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Met Ile Gln Ile Ser Asn Asp Leu
65                  70                  75                  80

Glu Asn Leu Arg Asp Leu Leu His Val Leu Ala Phe Ser Lys Ser Cys
                85                  90                  95

Ser Leu Pro Gln Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Gln Gln Leu Asp Leu Ser Pro
    130                 135                 140

Gly Cys
145

## Description

The present invention is in the field of human medicine, particularly in the treatment of obesity and disorders associated with obesity. More specifically, the present invention relates to anti-obesity proteins based on chimpanzee leptin.

Obesity, and especially upper body obesity, is a common and very serious public health problem in the United States and throughout the world. According to recent statistics, more than 25% of the United States population and 27% of the Canadian population are overweight. Kuczmarski, Amer. J. of Clin. Nutr. 55: 495S - 502S (1992); Reeder et. al., Can. Med. Ass. J., 23: 226-233 (1992). Upper body obesity is the strongest risk factor known for type II diabetes mellitus, and is a strong risk factor for cardiovascular disease and cancer as well. Recent estimates for the medical cost of obesity are $150,000,000,000 world wide. The problem has become serious enough that the surgeon general has begun an initiative to combat the ever increasing adiposity rampant in American society.

Much of this obesity induced pathology can be attributed to the strong association with dyslipidemia, hypertension, and insulin resistance. Many studies have demonstrated that reduction in obesity by diet and exercise reduces these risk factors dramatically. Unfortunately, these treatments are largely unsuccessful with a failure rate reaching 95%. This failure may be due to the fact that the condition is strongly associated with genetically inherited factors that contribute to increased appetite, preference for highly caloric foods, reduced physical activity, and increased lipogenic metabolism. This indicates that people inheriting these genetic traits are prone to becoming obese regardless of their efforts to combat the condition. Therefore, a pharmacological agent that can correct this adiposity handicap and allow the physician to successfully treat obese patients in spite of their genetic inheritance is needed.

The *ob /ob* mouse is a model of obesity and diabetes that is known to carry an autosomal recessive trait linked to a mutation in the sixth chromosome. Recently, Yiying Zhang and co-workers published the positional cloning of the mouse gene linked with this condition. Yiying Zhang et al. Nature 372: 425-32 (1994). This report disclosed the murine and human protein expressed in adipose tissue. Likewise, Murakami et al., in Biochemical and Biophysical Research Communications 209(3) :944-52 (1995) report the cloning and expression of the rat obese gene. The protein, which is encoded by the *ob* gene, has demonstrated an ability to effectively regulate adiposity in mice. Pelleymounter et al., Science 269: 540-543 (1995).

A parenteral formulation containing insoluble protein causes problems relating to inconsistency in the dose-response as well as unpredictability. The unpredictability is believed to be due to greater variability in the pharamacokinetics in suspension formulations. The insoluble formulations must first dissolve prior to adsorption. It is hypothesized that this step has significant varaibility in a subcutaneous depot.

Unfortunately, the naturally occurring obesity proteins demonstrate a propensity to aggregate and cause a response at the site of injection making the preparation of a soluble, pharmaceutically acceptable parenteral formulation exceedingly difficult. The molecular interactions amongst the preservative, buffer, ionic strength, pH, temperature, and any additional excipients such as a surfactant, or sugar are highly unpredictable in view of the propensity for the obesity protein to aggregate and precipitate from the formulation.

Obesity proteins have been developed and have demonstrated pharmacological activity. Some of these proteins demonstrate significant improvement in physical properties and stability. Such proteins are disclosed in WO96/23517.

The present invention provides obesity proteins based on the chimpanzee leptin sequence disclosed in U.S. application number 60/003,935 and conditions under which solubility of such proteins are enhanced. Thus, the invention provides longer shelf life, ease of manufacture, and more convenient patient delivery. Most unexpectedly, the physical stability of the formulation is greatly enhanced in the presence of alkyl parabens such as methylparaben, ethylparaben, propylparaben, and butylparaben, as well as chlorobutanol or a mixture thereof. That is, when formulated under the conditions described herein, these novel obesity proteins remain soluble at much higher concentrations and at a pH range acceptable for a soluble, multi-use parenteral formulation. Accordingly, the present invention provides soluble, parenteral formulations of novel obesity proteins based on chimpanzee leptin.

Proteins useful for treating obesity selected from SEQ ID NOs 1-13 are claimed. Soluble parenteral formulations, comprising an obesity protein and a preservative selected from the group consisting of alkylparaben, chlorobutanol, or a mixture thereof are also claimed.

For purposes of the present invention, as disclosed and claimed herein, the following terms and abbreviations are defined as follows:

Alkylparaben -- refers to a C₁ to C₄ alkylparaben. Preferably, alkylparaben is methylparaben, ethylparaben, propylparaben, or butylparaben.

Base pair (bp) -- refers to DNA or RNA. The abbreviations A,C,G, and T correspond to the 5'-monophosphate forms of the nucleotides (deoxy)adenine, (deoxy)cytidine, (deoxy) guanine, and (deoxy)thymine, respectively, when they occur in DNA molecules. The abbreviations U,C,G, and T correspond to the 5'-monophosphate forms of the nucleosides uracil, cytidine, guanine, and thymine, respectively when they occur in RNA molecules. In double stranded DNA, base pair may refer to a partnership of A with T or C with G. In a DNA/RNA heteroduplex, base pair may refer to a partnership of T with U or C with G.

Obesity protein -- refers to a protein of the Formula (I): 
(SEQ ID NO: 1)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Xaa Ser Val Ser Ser

35 40 45
Lys Gln Lys Val Thr Gly Leu Asp Phe Ile Pro Gly Leu His Pro Ile

50 55 60
 
Leu Thr Leu Ser Lys Met Asp Gln Thr Leu Ala Val Tyr Gln Gln Ile

65                  70                  75                  80
Leu Thr Ser Met Pro Ser Arg Asn Met Ile Gln Ile Ser Asn Asp Leu

                85                  90                  95
Glu Asn Leu Arg Asp Leu Leu His Val Leu Ala Phe Ser Lys Ser Cys

            100                 105                 110
His Leu Pro Trp Ala Ser Gly Leu Glu Thr Leu Asp Ser Leu Gly Gly

        115                 120                 125
Val Leu Glu Ala Ser Gly Tyr Ser Thr Glu Val Val Ala Leu Ser Arg

    130                 135                 140
Leu Gln Gly Ser Leu Gln Asp Met Leu Trp Gln Leu Asp Leu Ser Pro

145
Gly Cys                                                         (I)
 wherein:
Xaa at position 28 is Gln or absent;
said protein having at least one of the following substitutions:
Gln at position 4 is replaced with Glu;
Gln at position 7 is replaced with Glu;
Asn at position 22 is replaced with Gln or Asp;
Thr at position 27 is replaced with Ala;
Xaa at position 28 is replaced with Glu;
Gln at position 34 is replaced with Glu;
Met at position 54 is replaced with methionine sulfoxide, Leu, Ile, Val, Ala, or Gly;
Gln at position 56 is replaced with Glu;
Gln at position 62 is replaced with Glu;
Gln at position 63 is replaced with Glu;
Met at position 68 is replaced with methionine sulfoxide, Leu, Ile, Val, Ala, or Gly;
Asn at position 72 is replaced with Gln, Glu, or Asp;
Gln at position 75 is replaced with Glu;
Ser at position 77 is replaced with Ala;
Asn at position 78 is replaced with Gln or Asp;
Asn at position 82 is replaced with Gln or Asp;
His at position 97 is replaced with Gln, Asn, Ala, Gly, Ser, or Pro;
Trp at position 100 is replaced with Ala, Glu, Asp, Asn, Met, Ile, Phe, Tyr, Ser, Thr, Gly, Gln, Val or Leu;
Ala at position 101 is replaced with Ser, Asn, Gly, His, Pro, Thr, or Val;
Ser at position 102 is replaced with Arg;
Gly at position 103 is replaced with Ala;
Glu at position 105 is replaced with Gln;
Thr at position 106 is replaced with Lys or Ser;
Leu at position 107 is replaced with Pro;
Asp at position 108 is replaced with Glu;
Gly at position 111 is replaced with Asp;
Gly at position 118 is replaced with Leu;
Gln at position 130 is replaced with Glu;
Gln at position 134 is replaced with Glu;
Met at position 136 is replaced with methionine sulfoxide, Leu, Ile, Val, Ala, or Gly;
Trp at position 138 is replaced with Ala, Glu, Asp, Asn, Met, Ile, Phe, Tyr, Ser, Thr, Gly, Gln, Val or Leu; or
Gln at position 139 is replaced with Glu;
or a pharmaceutically acceptable salt thereof.

Plasmid -- an extrachromosomal self-replicating genetic element.

Reading frame -- the nucleotide sequence from which translation occurs "read" in triplets by the translational apparatus of tRNA, ribosomes and associated factors, each triplet corresponding to a particular amino acid. Because each triplet is distinct and of the same length, the coding sequence must be a multiple of three. A base pair insertion or deletion (termed a frameshift mutation) may result in two different proteins being coded for by the same DNA segment. To insure against this, the triplet codons corresponding to the desired polypeptide must be aligned in multiples of three from the initiation codon, i.e. the correct "reading frame" must be maintained. In the creation of fusion proteins containing a chelating peptide, the reading frame of the DNA sequence encoding the structural protein must be maintained in the DNA sequence encoding the chelating peptide.

Recombinant DNA Cloning Vector -- any autonomously replicating agent including, but not limited to, plasmids and phages, comprising a DNA molecule to which one or more additional DNA segments can or have been added.

Recombinant DNA Expression Vector -- any recombinant DNA cloning vector in which a promoter has been incorporated.

Replicon -- A DNA sequence that controls and allows for autonomous replication of a plasmid or other vector.

Transcription -- the process whereby information contained in a nucleotide sequence of DNA is transferred to a complementary RNA sequence.

Translation -- the process whereby the genetic information of messenger RNA is used to specify and direct the synthesis of a polypeptide chain.

Vector -- a replicon used for the transformation of cells in gene manipulation bearing polynucleotide sequences corresponding to appropriate protein molecules which, when combined with appropriate control sequences, confer specific properties on the host cell to be transformed. Plasmids, viruses, and bacteriophage are suitable vectors, since they are replicons in their own right. Artificial vectors are constructed by cutting and joining DNA molecules from different sources using restriction enzymes and ligases. Vectors include Recombinant DNA cloning vectors and Recombinant DNA expression vectors.

Treating -- as used herein, describes the management and care of a patient for the purpose of combating the disease, condition, or disorder and includes the administration of a protein of present invention to prevent the onset of the symptoms or complications, alleviating the symptoms or complications, or eliminating the disease, condition, or disorder. Treating as used herein includes the administration of the protein for cosmetic purposes. A cosmetic purpose seeks to control the weight of a mammal to improve bodily appearance.

Isotonicity agent -- isotonicity agent refers to an agent that is physiologically tolerated and embarks a suitable tonicity to the formulation to prevent the net flow of water across the cell membrane. Compounds, such as glycerin, are commonly used for such purposes at known concentrations. Other possible isotonicity agents include salts, e.g., NaCl, dextrose, and lactose.

Physiologically tolerated buffer -- a physiologically tolerated buffer is known in the art. A physiologically tolerated buffer is preferably a phosphate buffer, like sodium phosphate. Other physiologically tolerated buffers include TRIS, sodium acetate, sodium citrate as well as conventional glycine and arginine based buffering systems. The selection and concentration of buffer is known in the art.

The nucleotide and amino acid abbreviations are accepted by the United States Patent and Trademark Office as set forth in 37 C.F.R. § 1.822 (b) (2) (1993). One skilled in the art would recognize that certain amino acids are prone to rearrangement. For example, Asp may rearrange to aspartimide and isoasparigine as described in I. Schön et al., Int. J. Peptide Protein Res. 14: 485-94 (1979) and references cited therein. These rearrangement derivatives are included within the scope of the present invention. Unless otherwise indicated the amino acids are in the L configuration.

As noted above, the invention provides a soluble parenteral formulation, comprising a novel obesity protein and a preservative selected from the group consisting of alkylparaben or chlorobutanol. In the presence of these preservatives, the presently claimed obesity proteins unexpectedly remain in solution making a soluble, parenteral formulation possible.

A parenteral formulation must meet guidelines for preservative effectiveness to be a commercially viable product. Preservatives known in the art as being acceptable in parenteral formulations include: phenol, m-cresol, benzyl alcohol, methylparaben, chlorobutanol, p-cresol, phenylmercuric nitrate, thimerosal and various mixtures thereof. See, e.g., WALLHAUSER, K.-H., DEVELOP. BIOL. STANDARD. 24, pp. 9-28 (Basel, S. Krager, 1974).

Most unexpectedly, a select number of preservatives have been identified that provide good formulation stability These select preservatives are alkylparaben or chlorobutanol. Most preferrably, the preservative is methylparaben, ethylparaben, propylparaben, or butylparaben. Most unexpectedly, the obesity protein does not aggregate or precipitate in the presence of these preservatives at the conditions necessary to formulate and particularly conditions at 37°C.

The concentration of obesity protein in the formulation is about 1.0 mg/mL to about 100 mg/mL; preferably about 5.0 mg/mL to about 50.0 mg/mL; most preferably, about 10.0 mg/mL. The concentration of preservative required is the concentration necessary to maintain preservative effectiveness. The relative amounts of preservative necessary to maintain preservative effectiveness varies with the preservative used. Generally, the amount necessary can be found in WALLHAUSER, K.-H., DEVELOP. BIOL. STANDARD. 24, pp. 9-28 (Basel, S. Krager, 1974), herein incorporated by reference. The optimal concentration of the preservative depends on the preservative, its solubility, and the pH of the formulation.

An isotonicity agent, preferably glycerin, may be additionally added to the formulation. The concentration of the isotonicity agent is in the range known in the art for parenteral formulations, preferably about 16 mg/mL. The pH of the formulation may also be buffered with a physiologically tolerated buffer, preferably a phosphate buffer, like sodium phosphate. Other acceptable physiologically tolerated buffers include TRIS, sodium acetate, or sodium citrate. The selection and concentration of buffer is known in the art; however, the formulations of the present invention are preferably prepared in the absence of salt so that the ionic strength of the formulation is minimized.

Also included as optional embodiments are agents known to be synergistic with the preservative to provide enhanced antimicrobial effects. Such agents are recognized in the art and include for example EDTA, EGTA, citrate, and caprylic acid. The concentration of these agents varies with the desired preservation effect. A preferred agent is EDTA, particularly in conjunction with an alkylparabens at a concentration of about 0.025% to 0.4%.

The present formulations may optionally contain a physiologically tolerated solvent such as glycerol, propylene, glycol, phenoxy ethanol, phenyl ethyl alcohol. Such solvents are generally added to enhance the solubility of the protein in the preservation effectiveness of the formulation.

Other additives, such as a pharmaceutically acceptable solubilizers like Tween 20 (polyoxyethylene (20) sorbitan monolaurate), Tween 40 (polyoxyethylene (20) sorbitan monopalmitate), Tween 80 (polyoxyethylene (20) sorbitan monooleate), Pluronic F68 (polyoxyethylene polyoxypropylene block copolymers), BRIJ 35 (polyoxyethylene (23) lauryl ether), and PEG (polyethylene glycol) may optionally be added to the formulation to reduce aggregation. These additives are particularly useful if a pump or plastic container is used to administer the formulation. The presence of pharmaceutically acceptable surfactant mitigates the propensity for the protein to aggregate.

The parenteral formulations of the present invention can be prepared using conventional dissolution and mixing procedures. To prepare a suitable formulation, for example, a measured amount of human obesity protein in water is combined with the desired preservative in water in quantities sufficient to provide the protein and preservative at the desired concentration. The formulation is generally sterile filtered prior to administration. Variations of this process would be recognized by one of ordinary skill in the art. For example, the order the components are added, the surfactant used, the temperature and pH at which the formulation is prepared may be optimized for the concentration and means of administration used.

Preferably, the pH of the present formulations is about pH 7.0 to about 9.0 and, most preferably 7.6 to 8.5. The formulations are preferably prepared under basic conditions by mixing the obesity protein and preservative at a pH greater than pH 7.0. Preferably, the pH is about 7.6 to 8.5, and most preferably about pH 7.8. Ideally, a preservative and water solution is mixed at pH 7.6 to 8.5. Added to this solution is obesity protein in water. The pH is adjusted, as necessary, to about pH 7.6 to 8.5. The solution is then held until the components are dissolved, approximately 20 to 40 minutes, preferably about 30 minutes. The base used to adjust the pH of the formulation may be one or more pharmaceutically acceptable bases such as sodium hydroxide or potassium hydroxide. The preferred base is sodium hydroxide.

The formulations prepared in accordance with the present invention may be used in a syringe, injector, pumps or any other device recognized in the art for parenteral administration.

Preferred proteins of the present invention are those of Formula I, wherein:
Gln at position 4 is replaced with Glu;
Gln at position 7 is replaced with Glu;
Asn at position 22 is replaced with Gln or Asp;
Thr at position 27 is replaced with Ala;
Gln at position 28 is replaced with Glu;
Gln at position 34 is replaced with Glu;
Met at position 54 is replaced with methionine sulfoxide, Leu, or Ala;
Gln at position 56 is replaced with Glu;
Gln at position 62 is replaced with Glu;
Gln at position 63 is replaced with Glu;
Met at position 68 is replaced with methionine sulfoxide, or Leu;
Asn at position 72 is replaced with Gln or Asp;
Gln at position 75 is replaced with Glu;
Asn at position 78 is replaced with Gln or Asp;
Asn at position 82 is replaced with Gln or Asp;
Gln at position 130 is replaced with Glu;
Gln at position 134 is replaced with Glu;
Met at position 136 is replaced with methionine sulfoxide, Leu, Ile; or
Gln at position 139 is replaced with Glu.

Other preferred proteins are those of Formula I wherein:
Asn at position 22 is replaced with Gln or Asp;
Thr at position 27 is replaced with Ala;
Met at position 54 is replaced with methionine sulfoxide, Leu, or Ala;
Met at position 68 is replaced with methionine sulfoxide, or Leu;
Asn at position 72 is replaced with Gln or Asp;
Asn at position 78 is replaced with Gln or Asp;
Asn at position 82 is replaced with Gln or Asp; or
Met at position 136 is replaced with methionine sulfoxide, Leu, or Ile.

Still yet additional preferred proteins are those of Formula I, wherein:
Asn at position 22 is replaced with Gln or Asp;
Thr at position 27 is replaced with Ala;
Met at position 54 is replaced with Leu, or Ala;
Met at position 68 is replaced with Leu;
Asn at position 72 is replaced with Gln or Asp;
Asn at position 78 is replaced with Gln or Asp;
Asn at position 82 is replaced with Gln or Asp; or
Met at position 136 is replaced with Leu, or Ile.

Preferred species within Formula I include species of SEQ ID NO: 2 and SEO ID NO: 3:

(SEQ ID NO: 2)
                5                   10                  15
Val Pro Ile Gln Lys Val Gln Asp Asp Thr Lys Thr Leu Ile Lys Thr

            20                  25                  30
Ile Val Thr Arg Ile Asp Asp Ile Ser His Thr Gln Ser Val Ser Ser

        35                  40                  45
Lys Gln Lys Val Thr Gly Leu Asp Phe Ile Pro Gly Leu His Pro Ile

    50                  55                  60
Leu Thr Leu Ser Lys Met Asp Gln Thr Leu Ala Val Tyr Gln Gln Ile

65                  70                  75                  80
Leu Thr Ser Met Pro Ser Arg Asn Met Ile Gln Ile Ser Asn Asp Leu

                85                  90                  95
Glu Asn Leu Arg Asp Leu Leu His Val Leu Ala Phe Ser Lys Ser Cys

            100                 105                 110
His Leu Pro Trp Ala Ser Gly Leu Glu Thr Leu Asp Ser Leu Gly Gly

        115                 120                 125
Val Leu Glu Ala Ser Gly Tyr Ser Thr Glu Val Val Ala Leu Ser Arg

    130                 135                 140
Leu Gln Gly Ser Leu Gln Asp Met Leu Trp Gln Leu Asp Leu Ser Pro

145
Gly Cys

(SEQ ID NO: 3)
5 10 15
Val Pro Ile Gln Lys Val Gln Asp Asp Thr Lys Thr Leu Ile Lys Thr

20 25 30
Ile Val Thr Arg Ile Asn Asp Ile Ser His Ala Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Met Ile Gln Ile Ser Asn Asp Leu

85 90 95
Glu Asn Leu Arg Asp Leu Leu His Val Leu Ala Phe Ser Lys Ser Cys

100 105 110
His Leu Pro Trp Ala Ser Gly Leu Glu Thr Leu Asp Ser Leu Gly Gly

115
Val Leu Glu Ala Ser Gly Tyr Ser Thr Glu Val Val Ala Leu Ser Arg
120 125

130 135 140
Leu Gln Gly Ser Leu Gln Asp Met Leu Trp Gln Leu Asp Leu Ser Pro

145
Gly Cys

Most significantly, other preferred proteins of the present formulations are specific substitutions to amino acid residues 97 to 111, and/or 138 of the proteins of SEQ ID NO: 1. These substitutions result in additional stability and are superior therapeutic agents. These specific proteins are more readily formulated and are more pharmaceutically elegant, which results in superior delivery of therapeutic doses. Accordingly, preferred embodiments are formulation comprising proteins of the Formula II: 
(SEQ ID NO: 4)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Xaa Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Met Ile Gln Ile Ser Asn Asp Leu

85 90 95
Glu Asn Leu Arg Asp Leu Leu His Val Leu Ala Phe Ser Lys Ser Cys

100 105 110
His Leu Pro Trp Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Trp Gln Leu Asp Leu Ser Pro

145
Gly Cys (II)
 wherein:
Xaa at position 28 is Gln or absent;
said protein having at least one substitution selected from the group consisting of:
His at position 97 is replaced with Gln, Asn, Ala, Gly, Ser, or Pro;
Trp at position 100 is replaced with Ala, Glu, Asp, Asn, Met, Ile, Phe, Tyr, Ser, Thr, Gly, Gln, Val or Leu;
Ala at position 101 is replaced with Ser, Asn, Gly, His, Pro, Thr, or Val;
Ser at position 102 is replaced with Arg;
Gly at position 103 is replaced with Ala;
Glu at position 105 is replaced with Gln;
Thr at position 106 is replaced with Lys or Ser;
Leu at position 107 is replaced with Pro;
Asp at position 108 is replaced with Glu;
Gly at position 111 is replaced with Asp; or
Trp at position 138 is replaced with Ala, Glu, Asp, Asn, Met, Ile, Phe, Tyr, Ser, Thr, Gly, Gln, Val or Leu;
or a pharmaceutically acceptable salt thereof.

Preferred proteins are a protein of the Formula III: 
(SEQ ID NO: 5)

                5                   10                  15
Val Pro Ile Gln Lys Val Gln Asp Asp Thr Lys Thr Leu Ile Lys Thr

            20                  25                  30
Ile Val Thr Arg Ile Asn Asp Ile Ser His Thr Gln Ser Val Ser Ser

        35                  40                  45
Lys Gln Lys Val Thr Gly Leu Asp Phe Ile Pro Gly Leu His Pro Ile

    50                  55                  60
Leu Thr Leu Ser Lys Met Asp Gln Thr Leu Ala Val Tyr Gln Gln Ile

65                  70                  75                  80
Leu Thr Ser Met Pro Ser Arg Asn Met Ile Gln Ile Ser Asn Asp Leu

                85                  90                  95
Glu Asn Leu Arg Asp Leu Leu His Val Leu Ala Phe Ser Lys Ser Cys

            100                 105                 110
His Leu Pro Trp Ala Ser Gly Leu Glu Thr Leu Asp Ser Leu Gly Gly

        115                 120                 125
Val Leu Glu Ala Ser Gly Tyr Ser Thr Glu Val Val Ala Leu Ser Arg

    130                 135                 140
Leu Gln Gly Ser Leu Gln Asp Met Leu Trp Gln Leu Asp Leu Ser Pro

145
Gly Cys                                                    (III)
 said protein having at least one substitution selected from the group consisting of:
His at position 97 is replaced with Gln, Asn, Ala, Gly, Ser, or Pro;
Trp at position 100 is replaced with Ala, Glu, Asp, Asn, Met, Ile, Phe, Tyr, Ser, Thr, Gly, Gln, Val or Leu;
Ala at position 101 is replaced with Ser, Asn, Gly, His, Pro, Thr, or Val;
Ser at position 102 is replaced with Arg;
Gly at position 103 is replaced with Ala;
Glu at position 105 is replaced with Gln;
Thr at position 106 is replaced with Lys or Ser;
Leu at position 107 is replaced with Pro;
Asp at position 108 is replaced with Glu;
Gly at position 111 is replaced with Asp; or
Trp at position 138 is replaced with Ala, Glu, Asp, Asn, Met, Ile, Phe, Tyr, Ser, Thr, Gly, Gln, Val or Leu;
or a pharmaceutically acceptable salt thereof.

More preferred proteins of the Formula III are those wherein:
His at position 97 is replaced with Gln, Asn, Ala, Gly, Ser or Pro;
Trp at position 100 is replaced with Ala, Glu, Asp, Asn, Met, Ile, Phe, Tyr, Ser, Thr, Gly, Gln or Leu;
Ala at position 101 is replaced with Ser, Asn, Gly, His, Pro, Thr or Val;
Glu at position 105 is replaced with Gln;
Thr at position 106 is replaced with Lys or Ser;
Leu at position 107 is replaced with Pro;
Asp at position 108 is replaced with Glu;
Gly at position 111 is replaced with Asp; or
Trp at position 138 is replaced with Ala, Glu, Asp, Asn, Met, Ile, Phe, Tyr, Ser, Thr, Gly, Gln, Val or Leu.

Other preferred proteins of the Formula III are those wherein:
His at position 97 is replaced with Ser or Pro;
Trp at position 100 is replaced with Ala, Gly, Gln, Val, Ile, or Leu;
Ala at position 101 is replaced with Thr; or
Trp at position 138 is replaced with Ala, Ile, Gly, Gln, Val or Leu.

Yet still additional preferred proteins of the Formula III are those wherein:
His at position 97 is replaced with Ser or Pro;
Trp at position 100 is replaced with Ala, Gln or Leu;
Ala at position 101 is replaced with Thr; or
Trp at position 138 is replaced with Gln.

Most preferred species of the present invention are those proteins having a di-sulfide bond between Cys at position 96 and Cys at position 146. Examples of most preferred species include species of SEQ ID NO: 6-13: 
(SEQ ID NO: 6)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Met Ile Gln Ile Ser Asn Asp Leu

85 90 95
Glu Asn Leu Arg Asp Leu Leu His Val Leu Ala Phe Ser Lys Ser Cys

100 105 110
His Leu Pro Ala Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Trp Gln Leu Asp Leu Ser Pro

145
 Gly Cys said protein having a di-sulfide bond between Cys at position 96 and Cys at position 146; or pharmaceutically acceptable salts thereof. 
(SEQ ID NO: 7)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Met Ile Gln Ile Ser Asn Asp Leu

85 90 95
Glu Asn Leu Arg Asp Leu Leu His Val Leu Ala Phe Ser Lys Ser Cys

100 105 110
His Leu Pro Gln Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Trp Gln Leu Asp Leu Ser Pro

145
Gly Cys
 said protein having a di-sulfide bond between Cys at position 96 and Cys at position 146; or pharmaceutically acceptable salts thereof. 
(SEQ ID NO: 8)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Met Ile Gln Ile Ser Asn Asp Leu

85 90 95
 
Glu Asn Leu Arg Asp Leu Leu His Val Leu Ala Phe Ser Lys Ser Cys

                100                 105                 110
His Leu Pro Trp Ala Ser Gly Leu Glu Thr Leu Asp Ser Leu Gly Gly

        115                 120                 125
Val Leu Glu Ala Ser Gly Tyr Ser Thr Glu Val Val Ala Leu Ser Arg

    130                 135                 140
Leu Gln Gly Ser Leu Gln Asp Met Leu Gln Gln Leu Asp Leu Ser Pro

145
Gly Cys
 said protein having a di-sulfide bond between Cys at position 96 and Cys at position 146; or pharmaceutically acceptable salts thereof. 
(SEQ ID NO: 9)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Met Ile Gln Ile Ser Asn Asp Leu

85 90 95
Glu Asn Leu Arg Asp Leu Leu His Val Leu Ala Phe Ser Lys Ser Cys

100 105 110
His Leu Pro Gln Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Gln Gln Leu Asp Leu Ser Pro

145
Gly Cys
 said protein having a di-sulfide bond between Cys at position 96 and Cys at position 146; or pharmaceutically acceptable salts thereof. 
(SEQ ID NO: 10)
5 10 15
Val Pro Ile Gln Lys Val Gln Asp Asp Thr Lys Thr Leu Ile Lys Thr

20 25 30
Ile Val Thr Arg Ile Asn Asp Ile Ser His Ala Gln Ser Val Ser Ser

35 40 45
 
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Met Ile Gln Ile Ser Asn Asp Leu

85 90 95
Glu Asn Leu Arg Asp Leu Leu His Val Leu Ala Phe Ser Lys Ser Cys

100 105 110
His Leu Pro Ala Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Trp Gln Leu Asp Leu Ser Pro

145
Gly Cys
 said protein having a di-sulfide bond between Cys at position 96 and Cys at position 146; or pharmaceutically acceptable salts thereof. 
(SEQ ID NO: 11)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Met Ile Gln Ile Ser Asn Asp Leu

85 90 95
Glu Asn Leu Arg Asp Leu Leu His Val Leu Ala Phe Ser Lys Ser Cys

100 105 110
His Leu Pro Ala Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Gln Gln Leu Asp Leu Ser Pro

145
Gly Cys
 said protein having a di-sulfide bond between Cys at position 96 and Cys at position 146; or pharmaceutically acceptable salts thereof. 
(SEQ ID NO: 12)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Met Ile Gln Ile Ser Asn Asp Leu

85 90 95
Glu Asn Leu Arg Asp Leu Leu His Val Leu Ala Phe Ser Lys Ser Cys

100 105 110
Ser Leu Pro Gln Thr Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Gln Gln Leu Asp Leu Ser Pro

145
Gly Cys
 said protein having a di-sulfide bond between Cys at position 96 and Cys at position 146; or pharmaceutically acceptable salts thereof. 
(SEQ ID NO: 13)
                    5                   10                  15
Val Pro Ile Gln Lys Val Gln Asp Asp Thr Lys Thr Leu Ile Lys Thr

            20                  25                  30
Ile Val Thr Arg Ile Asn Asp Ile Ser His Thr Gln Ser Val Ser Ser

        35                  40                  45
Lys Gln Lys Val Thr Gly Leu Asp Phe Ile Pro Gly Leu His Pro Ile

    50                  55                  60
Leu Thr Leu Ser Lys Met Asp Gln Thr Leu Ala Val Tyr Gln Gln Ile

65                  70                  75                  80
Leu Thr Ser Met Pro Ser Arg Asn Met Ile Gln Ile Ser Asn Asp Leu

                85                  90                  95
Glu Asn Leu Arg Asp Leu Leu His Val Leu Ala Phe Ser Lys Ser Cys

            100                 105                 110
Ser Leu Pro Gln Ala Ser Gly Leu Glu Thr Leu Asp Ser Leu Gly Gly

        115                 120                 125
Val Leu Glu Ala Ser Gly Tyr Ser Thr Glu Val Val Ala Leu Ser Arg
 
    130                 135                 140
Leu Gln Gly Ser Leu Gln Asp Met Leu Gln Gln Leu Asp Leu Ser Pro

145
Gly Cys
 said protein having a di-sulfide bond between Cys at position 96 and Cys at position 146; or pharmaceutically acceptable salts thereof.

The obesity proteins of the present invention can be prepared by any of a variety of recognized peptide synthesis techniques including classical (solution) methods, solid phase methods, semi synthetic methods, and more recent recombinant DNA methods. Recombinant methods are preferred if a high yield is desired. The basic steps in the recombinant production of protein include:
a) construction of a synthetic or semi-synthetic (or isolation from natural sources) DNA encoding the obesity protein,
b) integrating the coding sequence into an expression vector in a manner suitable for the expression of the protein either alone or as a fusion protein,
c) transforming an appropriate eukaryotic or prokaryotic host cell with the expression vector, and
d) recovering and purifying the recombinantly produced protein.

Synthetic genes, the in vitro or in vivo transcription and translation of which will result in the production of the protein may be constructed by techniques well known in the art. Owing to the natural degeneracy of the genetic code, the skilled artisan will recognize that a sizable yet definite number of DNA sequences may be constructed which encode the desired proteins. In the preferred practice of the invention, synthesis is achieved by recombinant DNA technology.

Methodology of synthetic gene construction is well known in the art. For example, see Brown, et al. (1979) Methods in Enzymology, Academic Press, N.Y., Vol. 68, pgs. 109-151. The DNA sequence corresponding to the synthetic claimed protein gene may be generated using conventional DNA synthesizing apparatus such as the Applied Biosystems Model 380A or 380B DNA synthesizers (commercially available from Applied Biosystems, Inc., 850 Lincoln Center Drive, Foster City, CA 94404). It may be desirable in some applications to modify the coding sequence of the obesity protein so as to incorporate a convenient protease sensitive cleavage site, e.g., between the signal peptide and the structural protein facilitating the controlled excision of the signal peptide from the fusion protein construct.

The gene encoding the obesity protein may also be created by using polymerase chain reaction (PCR). The template can be a cDNA library (commercially available from CLONETECH or STRATAGENE) or mRNA isolated from the desired arrival adipose tissue. Such methodologies are well known in the art Maniatis, et al. Molecular Cloning: A Laboratory Manual, Cold Spring Harbor Press, Cold Spring Harbor Laboratory, Cold Spring Harbor, New York (1989).

The constructed or isolated DNA sequences are useful for expressing the obesity protein either by direct expression or as fusion protein. When the sequences are used in a fusion gene, the resulting product will require enzymatic or chemical cleavage. A variety of peptidases which cleave a polypeptide at specific sites or digest the peptides from the amino or carboxy termini (e.g. diaminopeptidase) of the peptide chain are known. Furthermore, particular chemicals (e.g. cyanogen bromide) will cleave a polypeptide chain at specific sites. The skilled artisan will appreciate the modifications necessary to the amino acid sequence (and synthetic or semi-synthetic coding sequence if recombinant means are employed) to incorporate site-specific internal cleavage sites. See U.S. Patent No. 5,126,249; Carter P., Site Specific Proteolysis of Fusion Proteins, Ch. 13 in Protein Purification: From Molecular Mechanisms to Large Scale Processes, American Chemical Soc., Washington, D.C. (1990).

Construction of suitable vectors containing the desired coding and control sequences employ standard ligation techniques. Isolated plasmids or DNA fragments are cleaved, tailored, and religated in the form desired to form the plasmids required.

To effect the translation of the desired protein, one inserts the engineered synthetic DNA sequence in any of a plethora of appropriate recombinant DNA expression vectors through the use of appropriate restriction endonucleases. A synthetic coding sequence may be designed to possess restriction endonuclease cleavage sites at either end of the transcript to facilitate isolation from and integration into these expression and amplification and expression plasmids. The isolated cDNA coding sequence may be readily modified by the use of synthetic linkers to facilitate the incorporation of this sequence into the desired cloning vectors by techniques well known in the art. The particular endonucleases employed will be dictated by the restriction endonuclease cleavage pattern of the parent expression vector to be employed. The restriction sites are chosen so as to properly orient the coding sequence with control sequences to achieve proper in-frame reading and expression of the protein.

In general, plasmid vectors containing promoters and control sequences which are derived from species compatible with the host cell are used with these hosts. The vector ordinarily carries a replication origin as well as marker sequences which are capable of providing phenotypic selection in transformed cells. For example, E. coli is typically transformed using pBR322, a plasmid derived from an E. coli species (Bolivar, et al., Gene 2: 95 (1977)). Plasmid pBR322 contains genes for ampicillin and tetracycline resistance and thus provides easy means for identifying transformed cells. The pBR322 plasmid, or other microbial plasmid must also contain or be modified to contain promoters and other control elements commonly used in recombinant DNA technology.

The desired coding sequence is inserted into an expression vector in the proper orientation to be transcribed from a promoter and ribosome binding site, both of which should be functional in the host cell in which the protein is to be expressed. An example of such an expression vector is a plasmid described in Belagaje et al., U.S. patent No. 5,304,493, the teachings of which are herein incorporated by reference. The gene encoding A-C-B proinsulin described in U.S. patent No. 5,304,493 can be removed from the plasmid pRB182 with restriction enzymes NdeI and BamHI. The isolated DNA sequences can be inserted into the plasmid backbone on a NdeI/BamHI restriction fragment cassette.

In general, prokaryotes are used for cloning of DNA sequences in constructing the vectors useful in the invention. For example, E. coli K12 strain 294 (ATCC No. 31446) is particularly useful. Other microbial strains which may be used include E. coli B and E. coli X1776 (ATCC No. 31537). These examples are illustrative rather than limiting.

Prokaryotes also are used for expression. The aforementioned strains, as well as E. coli W3110 (prototrophic, ATCC No. 27325), bacilli such as Bacillus subtilis, and other enterobacteriaceae such as Salmonella typhimurium or Serratia marcescans, and various pseudomonas species may be used. Promoters suitable for use with prokaryotic hosts include the β-lactamase (vector pGX2907 [ATCC 39344] contains the replicon and β-lactamase gene) and lactose promoter systems (Chang et al., Nature, 275:615 (1978); and Goeddel et al., Nature 281:544 (1979)), alkaline phosphatase, the tryptophan (trp) promoter system (vector pATH1 [ATCC 37695] is designed to facilitate expression of an open reading frame as a trpE fusion protein under control of the trp promoter) and hybrid promoters such as the tac promoter (isolatable from plasmid pDR540 ATCC-37282). However, other functional bacterial promoters, whose nucleotide sequences are generally known, enable one of skill in the art to ligate them to DNA encoding the protein using linkers or adaptors to supply any required restriction sites. Promoters for use in bacterial systems also will contain a Shine-Dalgarno sequence operably linked to the DNA encoding protein.

The DNA molecules may also be recombinantly produced in eukaryotic expression systems. Preferred promoters controlling transcription in mammalian host cells may be obtained from various sources, for example, the genomes of viruses such as: polyoma, Simian Virus 40 (SV40), adenovirus, retroviruses, hepatitis-B virus and most preferably cytomegalovirus, or from heterologous mammalian promoters, e.g. β-actin promoter. The early and late promoters of the SV40 virus are conveniently obtained as an SV40 restriction fragment which also contains the SV40 viral origin of replication. Fiers, et al., Nature, 273:113 (1978). The entire SV40 genome may be obtained from plasmid pBRSV, ATCC 45019. The immediate early promoter of the human cytomegalovirus may be obtained from plasmid pCMBb (ATCC 77177). Of course, promoters from the host cell or related species also are useful herein.

Transcription of the DNA by higher eukaryotes is increased by inserting an enhancer sequence into the vector. Enhancers are cis-acting elements of DNA, usually about 10-300 bp, that act on a promoter to increase its transcription. Enhancers are relatively oriented and positioned independently and have been found 5' (Laimins, L. et al., PNAS 78:993 (1981)) and 3' (Lusky, M. L., et al., Mol. Cell Bio. 3:1108 (1983)) to the transcription unit, within an intron (Banerji, J. L. et al., Cell 33:729 (1983)) as well as within the coding sequence itself (Osborne, T. F., et al., Mol. Cell Bio. 4:1293 (1984)). Many enhancer sequences are now known from mammalian genes (globin, RSV, SV40, EMC, elastase, albumin, alpha-fetoprotein and insulin). Typically, however, one will use an enhancer from a eukaryotic cell virus. Examples include the SV40 late enhancer, the cytomegalovirus early promoter enhancer, the polyoma enhancer on the late side of the replication origin, and adenovirus enhancers.

Expression vectors used in eukaryotic host cells (yeast, fungi, insect, plant, animal, human or nucleated cells from other multicellular organisms) will also contain sequences necessary for the termination of transcription which may affect mRNA expression. These regions are transcribed as polyadenylated segments in the untranslated portion of the mRNA encoding protein. The 3' untranslated regions also include transcription termination sites.

Expression vectors may contain a selection gene, also termed a selectable marker. Examples of suitable selectable markers for mammalian cells are dihydrofolate reductase (DHFR, which may be derived from the BglII/HindIII restriction fragment of pJOD-10 [ATCC 68815]), thymidine kinase (herpes simplex virus thymidine kinase is contained on the BamHI fragment of vP-5 clone [ATCC 2028]) or neomycin (G418) resistance genes (obtainable from pNN414 yeast artificial chromosome vector [ATCC 37682]). When such selectable markers are successfully transferred into a mammalian host cell, the transfected mammalian host cell can survive if placed under selective pressure. There are two widely used distinct categories of selective regimes. The first category is based on a cell's metabolism and the use of a mutant cell line which lacks the ability to grow without a supplemented media. Two examples are: CHO DHFR⁻ cells (ATCC CRL-9096) and mouse LTK⁻ cells (L-M(TK-) ATCC CCL-2.3). These cells lack the ability to grow without the addition of such nutrients as thymidine or hypoxanthine. Because these cells lack certain genes necessary for a complete nucleotide synthesis pathway, they cannot survive unless the missing nucleotides are provided in a supplemented media. An alternative to supplementing the media is to introduce an intact DHFR or TK gene into cells lacking the respective genes, thus altering their growth requirements. Individual cells which were not transformed with the DHFR or TK gene will not be capable of survival in nonsupplemented media.

The second category is dominant selection which refers to a selection scheme used in any cell type and does not require the use of a mutant cell line. These schemes typically use a drug to arrest growth of a host cell. Those cells which have a novel gene would express a protein conveying drug resistance and would survive the selection. Examples of such dominant selection use the drugs neomycin, Southern P. and Berg, P., J. Molec. Appl. Genet. 1: 327 (1982), mycophenolic acid, Mulligan, R. C. and Berg, P. Science 209:1422 (1980), or hygromycin, Sugden, B. et al., Mol Cell. Biol. 5:410-413 (1985). The three examples given above employ bacterial genes under eukaryotic control to convey resistance to the appropriate drug G418 or neomycin (geneticin), xgpt (mycophenolic acid) or hygromycin, respectively.

A preferred vector for eukaryotic expression is pRc/CMV. pRc/CMV is commercially available from Invitrogen Corporation, 3985 Sorrento Valley Blvd., San Diego, CA 92121. To confirm correct sequences in plasmids constructed, the ligation mixtures are used to transform E. coli K12 strain DH10B (ATCC 31446) and successful transformants selected by antibiotic resistance where appropriate. Plasmids from the transformants are prepared, analyzed by restriction and/or sequence by the method of Messing, et al., Nucleic Acids Res. 9:309 (1981).

Host cells may be transformed with the expression vectors of this invention and cultured in conventional nutrient media modified as is appropriate for inducing promoters, selecting transformants or amplifying genes. The culture conditions, such as temperature, pH and the like, are those previously used with the host cell selected for expression, and will be apparent to the ordinarily skilled artisan. The techniques of transforming cells with the aforementioned vectors are well known in the art and may be found in such general references as Maniatis, et al., Molecular Cloning: A Laboratory Manual, Cold Spring Harbor Press, Cold Spring Harbor Laboratory, Cold Spring Harbor, New York (1989), or Current Protocols in Molecular Biology (1989) and supplements.

Preferred suitable host cells for expressing the vectors encoding the claimed proteins in higher eukaryotes include: African green monkey kidney line cell line transformed by SV40 (COS-7, ATCC CRL-1651); transformed human primary embryonal kidney cell line 293, (Graham, F. L. et al., J. Gen Virol. 36:59-72 (1977), Virology 77:319-329, Virology 86:10-21); baby hamster kidney cells (BHK-21(C-13), ATCC CCL-10, Virology 16:147 (1962)); Chinese hamster ovary cells CHO-DHFR⁻ (ATCC CRL-9096), mouse Sertoli cells (TM4, ATCC CRL-1715, Biol. Reprod. 23:243-250 (1980)); African green monkey kidney cells (VERO 76, ATCC CRL-1587); human cervical epitheloid carcinoma cells (HeLa, ATCC CCL-2); canine kidney cells (MDCK, ATCC CCL-34); buffalo rat liver cells (BRL 3A, ATCC CRL-1442); human diploid lung cells (WI-38, ATCC CCL-75); human hepatocellular carcinoma cells (Hep G2, ATCC HB-8065);and mouse mammary tumor cells (MMT 060562, ATCC CCL51).

In addition to prokaryotes, unicellular eukaryotes such as yeast cultures may also be used. Saccharomyces cerevisiae, or common baker's yeast is the most commonly used eukaryotic microorganism, although a number of other strains are commonly available. For expression in Saccharomyces, the plasmid YRp7, for example, (ATCC-40053, Stinchcomb, et al., Nature 282:39 (1979); Kingsman et al., Gene 7:141 (1979); Tschemper et al., Gene 10:157 (1980)) is commonly used. This plasmid already contains the trp gene which provides a selection marker for a mutant strain of yeast lacking the ability to grow in tryptophan, for example ATCC no. 44076 or PEP4-1 (Jones, Genetics 85:12 (1977)).

Suitable promoting sequences for use with yeast hosts include the promoters for 3-phosphoglycerate kinase (found on plasmid pAP12BD ATCC 53231 and described in U.S. Patent No. 4,935,350, June 19, 1990) or other glycolytic enzymes such as enolase (found on plasmid pACl ATCC 39532), glyceraldehyde-3-phosphate dehydrogenase (derived from plasmid pHcGAPC1 ATCC 57090, 57091), zymomonas mobilis (United States Patent No. 5,000,000 issued March 19, 1991), hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which contain inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein (contained on plasmid vector pCL28XhoLHBPV ATCC 39475, United States Patent No. 4,840,896), glyceraldehyde 3-phosphate dehydrogenase, and enzymes responsible for maltose and galactose (GAL1 found on plasmid pRyl21 ATCC 37658) utilization. Suitable vectors and promoters for use in yeast expression are further described in R. Hitzeman et al., European Patent Publication No. 73,657A. Yeast enhancers such as the UAS Gal from Saccharomyces cerevisiae (found in conjunction with the CYC1 promoter on plasmid YEpsec--hIlbeta ATCC 67024), also are advantageously used with yeast promoters.

The following examples and preparations are provided merely to further illustrate the preparation of the formulations of the invention. The scope of the invention is not construed as merely consisting of the following examples.

### Example 1

### Obesity Protein Formulation

To generate a solution of a protein of SEQ ID NO: 6 (hereinafter, Protein NO: 6) lyophilized solid material is first dissolved in water to generate a stock solution (stock 1). The concentration of Protein NO: 6 in stock 1 is verified by UV/Vis spectrophometry using the known extinction coefficient for Protein NO: 6 at the maximum spectral absorbance (279nm or 280nm), measuring the maximum spectral absorbance (279nm or 280nm), and utilizing the dilution factor. A stock preservative solution containing methylparaben, for example, is prepared by dissolving the solid in water (stock 2). A solution of Protein NO: 6 at 1.6 mg/mL is prepared by addition of an aliquot of stock 1 to a container which held an aliquot of stock 2 along with the required quantity of water, at an alkaline pH (7.8±0.3); adjusted if necessary with HCl or NaOH. After an appropriate incubation period at room temperature (30 minutes), the solution pH is examined and adjusted if necessary with trace µL quantities of HCl or NaOH, to yield Protein NO: 6 solution at 1.6 mg/mL with 0.17 % methylparaben pH 7.8±0.1. The solution is then hand-filtered using a glass syringe with an attached 0.22µm syringe filter into a glass vial.

### Example 2

### Obesity Protein Formulation

To generate a solution of a protein of SEQ ID NO: 6 (hereinafter Protein NO: 6), lyophilized solid material is first dissolved in water to generate a stock solution (stock 1). The concentration of Protein NO: 6 in stock 1 is verified by UV/Vis Spectrophometry using the known extinction coefficient for Protein NO: 6 at the maximum spectral absorbance (279nm or 280nm), measuring the maximum spectral absorbance (279nm or 280nm), and utilizing the dilution factor. A stock preservative solution containing chlorobutanol, for example, is prepared by dissolving the solid in water (stock 2). A solution of Protein NO: 6 at 1.6 mg/mL is prepared by addition of an aliquot of stock 1 to a container which held an aliquot of stock 2 along with the required quantity of water, at an alkaline pH (7.8±0.3); adjusted if necessary with HCl or NaOH. After an appropriate incubation period at room temperature (30 minutes), the solution pH is examined and adjusted if necessary with trace µL quantities of HCl or NaOH, to yield an Protein NO: 6 solution at 1.6 mg/mL with 0.50% chlorobutanol pH 7.8±0.1. The solution is then hand-filtered using a glass syringe with an attached 0.22µm syringe filter into a glass vial.

### Example 3

### Obesity Protein Formulation

To generate a solution of a protein of SEQ ID NO: 6 (hereinafter Protein NO: 6), solid bulk material, lyophilized from a neutral water solution, is redissolved in water to generate a stock solution (stock 1). The concentration of Protein NO: 6 in stock 1 is verified by UV/Vis Spectrophometry by multiplying the maximum spectral absorbance (279nm or 280nm) by the dilution factor divided by the known extinction coefficient for Protein NO: 6. A stock preservative solution containing methylparaben is prepared by dissolving the solid in water (stock 2). A stock of an isotonicity agent, such as glycerin, is prepared by dissolving the neat liquid in water (stock 3). A stock of a physiologically-tolerated buffer, such as sodium phosphate is prepared by dissolving the solid in water (stock 4). A solution of Protein NO: 6 at 1.6 mg/mL is prepared by addition of an aliquot of stock 1 to a container which held an aliquot of stock 2 along with an aliquot of stock 3 along with the required quantity of water, adjusted if necessary with HCl or NaOH to an alkaline pH (7.8±0.3). After an appropriate incubation period at room temperature (30 minutes) an aliquot of stock 4 is added. The solution pH is then readjusted if necessary with trace µL quantities of HCl or NaOH, to yield an Protein NO: 6 solution,.for example, at 1.6 mg/mL with 0.17% methylparaben, 16 mg/mL glycerin, and 14mM sodium phosphate at pH 7.8±0.1. The solution is then hand-filtered using a glass syringe with an attached 0.22µm syringe filter into a glass vial.

### Example 4

### Obesity Protein Formulation

To generate a solution of Human Obesity Protein mimetic (Protein NO: 6), solid bulk material, lyophilized from a neutral water solution, is redissolved in water to generate a stock solution (stock 1). The concentration of Protein NO: 6 in stock 1 is verified by UV/Vis Spectrophometry by multiplying the maximum spectral absorbance (279nm or 280nm) by the dilution factor divided by the known extinction coefficient for Protein NO: 6. A stock preservative solution containing chlorobutanol, for example, is prepared by dissolving the solid in water (stock 2). A stock of an isotonicity agent, such as glycerin, is prepared by dissolving the neat liquid in water (stock 3). A stock of a physiologically-tolerated buffer, such as sodium phosphate is prepared by dissolving the solid in water (stock 4). A solution of Protein NO: 6 at 1.6 mg/mL is prepared by addition of an aliquot of stock 1 to a container which held an aliquot of stock 2 along with an aliquot of stock 3 along with the required quantity of water, adjusted if necessary with HCl or NaOH to an alkaline pH (7.8±0.3). After an appropriate incubation period at room temperature (30 minutes) an aliquot of stock 4 is added. The solution pH is then readjusted if necessary with trace µL quantities of HCl or NaOH, to yield an Protein NO: 6 solution,.for example, at 1.6 mg/mL with 0.5% chlorobutanol, 16 mg/mL glycerin, and 14mM sodium phosphate at pH 7.8±0.1. The solution is then hand-filtered using a glass syringe with an attached 0.22um syringe filter into a glass vial.

The principles, preferred embodiments and modes of operation of the present invention have been described in the foregoing specification. The invention which is intended to be protected herein, however, is not to be construed as limited to the particular forms disclosed, since they are to be regarded as illustrative rather than restrictive. Variations and changes may be made by those skilled in the art without departing from the spirit of the invention.

## Claims

1. An obesity protein of the formula: 
5                   10                  15
Val Pro Ile Gln Lys Val Gln Asp Asp Thr Lys Thr Leu Ile Lys Thr

            20                  25                  30
Ile Val Thr Arg Ile Asn Asp Ile Ser His Thr Xaa Ser Val Ser Ser

        35                  40                  45
Lys Gln Lys Val Thr Gly Leu Asp Phe Ile Pro Gly Leu His Pro Ile

    50                  55                  60
Leu Thr Leu Ser Lys Met Asp Gln Thr Leu Ala Val Tyr Gln Gln Ile

65                  70                  75                  80
Leu Thr Ser Met Pro Ser Arg Asn Met Ile Gln Ile Ser Asn Asp Leu

                85                  90                  95
Glu Asn Leu Arg Asp Leu Leu His Val Leu Ala Phe Ser Lys Ser Cys

            100                 105                 110
His Leu Pro Trp Ala Ser Gly Leu Glu Thr Leu Asp Ser Leu Gly Gly

        115                 120                 125
Val Leu Glu Ala Ser Gly Tyr Ser Thr Glu Val Val Ala Leu Ser Arg

    130                 135                 140
Leu Gln Gly Ser Leu Gln Asp Met Leu Trp Gln Leu Asp Leu Ser Pro

145
Gly Cys                 (SEQ ID NO: 1)
 wherein:
Xaa at position 28 is Gln or absent;
said protein having at least one of the following substitutions:
Gln at position 4 is replaced with Glu;
Gln at position 7 is replaced with Glu;
Asn at position 22 is replaced with Gln or Asp;
Thr at position 27 is replaced with Ala;
Xaa at position 28 is replaced with Glu;
Gln at position 34 is replaced with Glu;
Met at position 54 is replaced with methionine sulfoxide, Leu, Ile, Val, Ala, or Gly;
Gln at position 56 is replaced with Glu;
Gln at position 62 is replaced with Glu;
Gln at position 63 is replaced with Glu;
Met at position 68 is replaced with methionine sulfoxide, Leu, Ile, Val, Ala, or Gly;
Asn at position 72 is replaced with Gln, Glu, or Asp;
Gln at position 75 is replaced with Glu;
Ser at position 77 is replaced with Ala;
Asn at position 78 is replaced with Gln or Asp;
Asn at position 82 is replaced with Gln or Asp;
His at position 97 is replaced with Gln, Asn, Ala, Gly, Ser, or Pro;
Trp at position 100 is replaced with Ala, Glu, Asp, Asn, Met, Ile, Phe, Tyr, Ser, Thr, Gly, Gln, Val or Leu;
Ala at position 101 is replaced with Ser, Asn, Gly, His, Pro, Thr, or Val;
Ser at position 102 is replaced with Arg;
Gly at position 103 is replaced with Ala;
Glu at position 105 is replaced with Gln;
Thr at position 106 is replaced with Lys or Ser;
Leu at position 107 is replaced with Pro;
Asp at position 108 is replaced with Glu;
Gly at position 111 is replaced with Asp;
Gly at position 118 is replaced with Leu;
Gln at position 130 is replaced with Glu;
Gln at position 134 is replaced with Glu;
Met at position 136 is replaced with methionine sulfoxide, Leu, Ile, Val, Ala, or Gly;
Trp at position 138 is replaced with Ala, Glu, Asp, Asn, Met, Ile, Phe, Tyr, Ser, Thr, Gly, Gln, Val or Leu; or
Gln at position 139 is replaced with Glu;
or a pharmaceutically acceptable salt thereof.

2. An obesity protein of Claim 1 selected from the group consisting of SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, and SEQ ID NO: 13.

3. A soluble parenteral formulation, comprising an obesity protein of Claim 1 and a preservative selected from the group consisting of alkylparaben, chlorobutanol, or a mixture thereof.

4. A formulation of Claim 3, wherein the preservative is methylparaben, ethylparaben, propylparaben, or butylparaben.

5. A formulation of Claim 4, wherein the concentration of obesity protein is about 1.0 mg/mL to about 100 mg/mL.

6. A formulation of Claim 4, wherein the concentration of obesity protein is about 5.0 mg/mL to about 50.0 mg/mL.

7. A formulation of Claim 6, which further comprises an isotonicity agent.

8. A formulation of Claim 7, which further comprises a physiologically acceptable buffer.

9. A formulation of Claim 8, wherein the concentration of obesity protein is about 10 mg/mL, the preservative is methylparaben, the isotonicity agent is glycerin at about 16 mg/mL, and the physiologically acceptable buffer is disodium phosphate at about 14 mM, and which further comprises about 0.1% EDTA phosphate.

10. A formulation of any one of Claims 3 through 9, wherein the obesity protein is a protein of SEQ ID NO: 2: 
(SEQ ID NO: 2)

                    5                   10                  15
Val Pro Ile Gln Lys Val Gln Asp Asp Thr Lys Thr Leu Ile Lys Thr

            20                  25                  30
Ile Val Thr Arg Ile Asp Asp Ile Ser His Thr Gln Ser Val Ser Ser

        35                  40                  45
Lys Gln Lys Val Thr Gly Leu Asp Phe Ile Pro Gly Leu His Pro Ile

    50                  55                  60
Leu Thr Leu Ser Lys Met Asp Gln Thr Leu Ala Val Tyr Gln Gln Ile

65                  70                  75                  80
Leu Thr Ser Met Pro Ser Arg Asn Val Ile Gln Ile Ser Asn Asp Leu

                85                  90                  95
Glu Asn Leu Arg Asp Leu Leu His Val Leu Ala Phe Ser Lys Ser Cys

            100                 105                 110
His Leu Pro Trp Ala Ser Gly Leu Glu Thr Leu Asp Ser Leu Gly Gly

        115                 120                 125
Val Leu Glu Ala Ser Gly Tyr Ser Thr Glu Val Val Ala Leu Ser Arg

    130                 135                 140
Leu Gln Gly Ser Leu Gln Asp Met Leu Trp Gln Leu Asp Leu Ser Pro

145
Gly Cys
 said protein having a di-sulfide bond between Cys at position 96 and Cys at position 146; or pharmaceutically acceptable salts thereof.

11. A formulation of any one of Claims 3 through 9, wherein the obesity protein is a protein of SEQ ID NO: 6: 
(SEQ ID NO: 6)

                5                   10                  15
Val Pro Ile Gln Lys Val Gln Asp Asp Thr Lys Thr Leu Ile Lys Thr

            20                  25                  30
Ile Val Thr Arg Ile Asn Asp Ile Ser His Thr Gln Ser Val Ser Ser

        35                  40                  45
Lys Gln Lys Val Thr Gly Leu Asp Phe Ile Pro Gly Leu His Pro Ile

    50                  55                  60
 
Leu Thr Leu Ser Lys Met Asp Gln Thr Leu Ala Val Tyr Gln Gln Ile

65                  70                  75                  80
Leu Thr Ser Met Pro Ser Arg Asn Met Ile Gln Ile Ser Asn Asp Leu

                85                  90                  95
Glu Asn Leu Arg Asp Leu Leu His Val Leu Ala Phe Ser Lys Ser Cys

            100                 105                 110
His Leu Pro Ala Ala Ser Gly Leu Glu Thr Leu Asp Ser Leu Gly Gly

        115                 120                 125
Val Leu Glu Ala Ser Gly Tyr Ser Thr Glu Val Val Ala Leu Ser Arg

    130                 135                 140
Leu Gln Gly Ser Leu Gln Asp Met Leu Trp Gln Leu Asp Leu Ser Pro

145
Gly Cys
 said protein having a di-sulfide bond between Cys at position 96 and Cys at position 146; or pharmaceutically acceptable salts thereof.

12. Use of a protein of Claim 1 for the manufacture of a medicament for the treatment of obesity.

13. Use of a protein selected from the group consisting of SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, and SEQ ID NO: 13 for the manufacture of a medicament for the treatment of obesity.
